# EUROPEAN PATENT APPLICATION

(11) **EP 3 028 698 A1**
(43) Date of publication of application: **08.06.2016**
(21) Application number: 14831923.9
(22) Date of filing: 24.07.2014
(51) Int. Cl.: A61K 31/407, A61K 9/24, A61P 29/00

(54) **THREE-PHASE SYSTEM FOR MODIFIED RELEASE OF A NON-STEROIDAL ANTIINFLAMMATORY**

(30) Priority: 02.08.2013 MX 2013008993
(71) Applicant: Laboratorio Raam de Sahuayo, S.a. de C.V., 59000 Sahuayo, Michoacán (MX)
(72) Inventor: AMEZCUA AMEZCUA, Federico, 59000 Sahuayo, MICH. (MX); COVARRUBIAS PINEDO, Amador, 45010 Zapopan, JAL. (MX)
(74) Representative: Balder IP Law, S.L.
(86) International application number: PCT/MX2014/000116
(87) International publication number: WO 2015/016697

(57) **Abstract**

The present invention relates to a novel pharmaceutical composition in solid form of a nonsteroidal anti-inflammatory and/or pharmaceutically acceptable salts thereof, consisting in a tablet comprising a modified delivery system in three phases, which are: a) a delayed-release core, characterized by being localized at the center of the composition; b) an immediate-release underlayer, which is characterized by fully and/or partially covering the core; and, c) an extended-release top layer, which is characterized by fully and/or partially covering the core. Said composition is orally administered and is used to treat mild to moderate pain caused by headache, myalgia, altralgia, eye pain, photophobia, burning feeling secondary to eye surgery, postoperative pain, neuropathic pain, swelling, among others.

## Description

### Field of the Invention

The present invention relates to a pharmaceutical composition in solid form for oral administration which comprises a modified release system of a nonsteroidal anti-inflammatory and/or their pharmaceutically acceptable salts, consisting of three phases: a) at least one centric core with delayed release; b) an underlayer of immediate release; c) an extended-release top layer; said composition is useful for the prevention and/or treatment of pain as altralgia, myalgia, eye pain, photophobia, burning feeling secondary to eye surgery, postoperative pain, neuropathic pain, swelling, headache, among others.

### Background

According to the International Association for the Study of Pain, it is defined as an unpleasant sensory and emotional experience associated with actual or potential tissue damage. Pain perception includes a sensory neuronal system (nociceptors) and afferent nerve pathways that respond to nociceptive tisue stimuli; however, nociception may be influenced by other factors, i.e., psychological (Puebla F, 2005).

For pharmacological treatment of pain, three categories of drugs are taking into account: Nonsteroidal antiinflammatory drugs (NSAIDs), opioid analgesics, and adjuvant analgesics.

Nonsteroidal antiinflammatory drugs (NSAIDs): They are considered as first choice treatment and are suitable for mild to moderate pain. NSAIDs, when used for the treatment of moderate to severe pain secondary to surgery by having properties to reduce the opioid dose, decrease the effects related to the use thereof. Making hematologic, gastrointestinal and kidney tests should be considered before using NSAIDs. NSAIDs include: aspirin, sulindac, diclofenac, etodolac, fenoprofen, ibuprofen, indomethacin, ketoprofen, ketorolac, meclofenamate, mefenamic acid, naproxen, acetaminophen, COX2 inhibitors, among others.

Opioid analgesics: they are used to treat moderate to severe pain of various etiologies. If the pain is not adequately controlled with NSAIDs, or pain is moderate to severe, an appropriate opioid should be added to the treatment with NSAIDs. Short-acting opioids should be dosed gradually first, before considering extended-release preparations. Within these are morphine, meperidine, ketamine, hydromorphone, oxycodone, methadone, levorphanol, fentanyl, codeine, nalbuphine, tramadol, among others.

Adjuvant analgesics: they are used to complement treatment with NSAIDs and opioids; should not be used alone in the treatment of acute pain. They reduce opioid and NSAIDs dosage requirements and speed up recovery. Among the adjuvant analgesics, the following may be mentioned: tricyclic antidepressants, antihistamines, benzodiazepines, caffeine, dextroamphetamine and clonidine (Gutiérrez A, 2007).

Ketorolac belongs to the family of nonsteroidal antiinflammatory drugs (NSAIDs), within the sub-classification of arylpropionic. Ketorolac shares the following structural characteristics with the drugs of its kind. 1) carboxylic acid group which binds the to site of action, 2) phenyl group, and 3) planar structure formed by a nitrogen which provides stability in binding to the cyclooxygenase (COX), an enzyme complex on which it acts as other NSAIDs.

Cyclooxygenase is an enzyme that has two different isoforms (COX-1 and COX-2) and is responsible for synthesizing prostaglandins (PGs) from arachidonic acid. Tissues in which each isoform is expressed are different: COX-1 is a constitutive part of most cells in the body, among which the parietal, heart, kidney and epitelial cells stand-out and is intended to synthesize PG protective of the gastric mucosa, the electrolytic homeostasis in cases of hypovolemia, PG involved in vasodilation and platelet aggregation facilitators. Furthermore COX-2 as well as being constitutive to alesser extent, is also induced by inflammatory processes, since besides prostaglandin synthesis, it is a catalyst for the production of prostacyclins and thromboxanes responsible for vasoconstriction/vasodilatation, fibrinolysis, sensitization of peripheral nociceptive receptors, and other events that occur during inflammation. Ketorolac, not being selective for any of the isoforms, inhibits both the formation of pro-inflammatory PGs and PGs in peripheral nociceptors (anti-inflammation and analgesia: desired pharmacological effects) such as production of PG "protective" from COX-1. This phenomenon explains many of the adverse side effects secondary to the administration of ketorolac (Zavaleta M. 2007).

Ketorolac tromethamine is rapidly and completely absorbed orally and parenterally. Its analgesic effect appears in the first half hour of intramuscular administration, peaks at 1-2 hours and lasts for 4-6 hours. The maximum plasma concentration, 2.2 □g/mL, after intramuscular administration, appears 50 minutes after a single dose of 30 mg, and 5.4 minutes after intravenous administration and 3 hours after oral administration, either in tablets or capsules (Sumary of Product Characteristics, Barcelona 2008).

In terms of distribution, ketorolac binds to plasma proteins by 99%; however this union is weak, so concomitant use of any other NSAID may displace ketorolac, increasing the plasma concentration of free drug and enhancing the adverse gastrointestinal effects. Once ketorolac has reached blood stream, the drug is metabolized via the liver resulting in inactive metabolites, mainly by conjugation (21%).

Ketorolac is renal, and excreted either as the drug without changes (60.2%), as a conjugated metabolite (21.9%), or hydroxylated (11.5%) (Zavaleta M. 2007).

In the prior art were found patents about sublingual compositions with a concentration of 10% to 15% of the composition (US7879901); a core, an active ingredient layer, a protective layer and an enteric coating (JP2004035535); sublingual dosage forms with an amount of active ingredient from 30% to 50% by weight (MX9606354); compositions in body fluid form, a matrix of fast dissolution and controled-release bio-bounded microparticles (EP0251680); ketorolac inert-coated microspheres, which may be coated with one or multiple layers to improve their aesthetics, performance, stability or taste (MX296317); cronotherapeutic rectangular tablet wherein the core is compressed by at least two superimposed layers and a retardation layer (US20040137062); among other.

### Object of the Invention

The object of the invention to provide a pharmaceutical composition of at least one nonsteroidal anti-inflammatory and/or pharmaceutically acceptable salts thereof, which comprises a triple delivery system, characterized by having three phases which are: a) a central core with delayed release; b) an underlayer of immediate release; c) an extended-release top layer; wherein said composition is useful in the treatment of pain, altralgia, myalgia, eye pain, photophobia, burning feeling secondary to eye surgery, postoperative pain, neuropathic pain, swelling, headache, among others.

### Detailed Description of the Invention

The present invention relates to a novel pharmaceutical composition in solid form that contains a nonsteroidal anti-inflammatory, which is ketorolac, and/or its pharmaceutically acceptable salts; and it is caracterised by having a triple release system, which consists of three phases, namely: a) at least one extended-release central core, which contains ketorolac and/or pharmaceutically acceptable salts thereof; and one or more pharmaceutically acceptable excipients, characterized by being delayed release in addition to being located in the center of the composition; b) an underlayer with ketorolac and/ or pharmaceutically acceptable salts thereof, and one or more pharmaceutically acceptable excipients; characterized by being immediate release; and, c) at least one top layer with ketorolac and/or pharmaceutically acceptable salts thereof, which is characterized by being extended release. The present composition is useful in the treatment of pain, altralgia, myalgia, eye pain, photophobia, burning feeling secondary to eye surgery, postoperative pain, neuropathic pain, swelling, headache, and other related disorders.

In the composition described herein, preferably the non-steroidal anti-inflammatory ketorolac and/or their pharmaceutically acceptable salts are used in the range of 2 mg to 100 mg, and more preferably in a range of 10 mg to 50 mg.

The delayed-release central core comprises one or more pharmaceutically acceptable excipients selected from diluents, and/or release modifiers, and/or binders, and/or lubricants, and/or solvents, among others.

The diluent contained in the delayed-release central core is selected from the following group: calcium lactate, and/or lactose, and/or microcrystalline cellulose, and/or sodium carboxymethylcellulose, and/or polydextrose, and/or sorbitol, and/or corn starch and its derivatives, and/or sucrose, and/or talc, and/or trehalose, and/or povidone, and/or combinations thereof.

The release modifiers contained in the delayed-release central core are selected from the following group: gum arabic, and/or acetyl tributyl citrate, and/or agar, and/or mannitol, and/or methylcellulose, and/or methylcellulose metolose, and/or pectin, and/or polycarbophil, and/or derivatives of methacrylate,s and/or combinations thereof.

Binders used in the extended-release central core are selected from the following group: alginic acid, and/or calcium carbonate, and/or calcium lactate, and/or carbopol, and/or carboxymethylcellulose calcium, and/or ceratonia, and/or chitosan, and/or copovidone, and/or dextrose, and/or ethyl cellulose, and/or guar gum, and/or hydroxyethyl methyl cellulose, and/or hydroxypropyl cellulose, and/or hypromellose, and/or anhydrous lactose, and/or sodium alginate, and/or polyvinylpyrrolidone, and/or starch, and/or sucrose, and/or combinations thereof.

Lubricants used in the delayed-release central core are selected from the following group: calcium phosphate, and/or calcium stearate, and/or hydrogenated castor oil, and/or glyceryl behenate, and/or glyceryl monostearate, and/or lauric acid, and/or leucine, and/or magnesium stearate, and/or myristic acid, and/or polyethylene glycol, and/or polyvinyl alcohol, and/or sodium hyaluronate, and/or sodium lauryl sulfate, and/or sodium stearyl fumarate, and/or stearic acid, and/or talc, and/or hydrogenated vegetable oil, and/or combinations thereof.

Solvents can be polar and/or non-polar, used for manufacturing the delayed-release central core, and are selected from the following group: acetone, and/or ethanol, and/or water, and/or benzyl alcohol, and/or combinations thereof.

The delayed-release central core is characterized because it may optionally contain at least one coating of a sensory layer comprising at least one sweetener, and/or flavoring agent (spicy, salty, sweet, bitter), and/or dye, and/or effervescent agents, and/or a polymer coating, as may be cellulose derivatives and/or methacrylic acid derivatives, and/or at least one plasticizer such as: polyethylene glycol derivatives and/or triacetin derivatives, and/or at least one solvent (water and/or alcohol) and/or a pharmaceutically acceptable excipient.

The immediate-release underlayer comprises one or more pharmaceutically acceptable excipients selected from diluents, and/or disintegrants, and/or binders, and/or lubricants, and/or solvents, among others.

The diluent contained in the immediate-release underlayer is selected from the following group: calcium lactate, and/or lactose, and/or microcrystalline cellulose, and/or sodium carboxymethylcellulose, and/or polydextrose, and/or sorbitol, and/or corn starch, and/or sucrose, and/or talc, and/or trehalose, and/or povidone, and/or combinations thereof.

The disintegrant contained in the immediate-release underlayer is selected from the following group: alginic acid, and/or calcium alginate, and/or carboxymethylcellulose, and/or microcrystalline cellulose, and/or silica, and/or corn starch, and/or croscarmellose, and/or crospovidone, and/or hydroxypropylcellulose, and/or povidone, and/or combinations thereof.

Binders contained in the immediate-release underlayer are selected from the following group: alginic acid, and/or calcium carbonate, and/or calcium lactate, and/or carbopol, and/or carboxymethylcellulose calcium, and/or ceratonia, and/or chitosan, and/or copovidone, and/or dextrose, and/or ethyl cellulose, and/or guar gum, and/or hydroxyethyl methyl cellulose, and/or hydroxypropyl cellulose, and/or hypromellose, and/or anhydrous lactose, and/or sodium alginate, and/or polyvinylpyrrolidone,, and/or starch, and/or sucrose, and/or combinations thereof.

Lubricants contained in the immediate-release underlayer are selected from the following group: calcium phosphate, and/or calcium stearate, and/or hydrogenated castor oil, and/or glyceryl behenate, and/or glyceryl monostearate, and/or lauric acid, and/or leucine, and/or magnesium stearate, and/or myristic acid, and/or polyethylene glycol, and/or polyvinyl alcohol, and/or sodium hyaluronate, and/or sodium lauryl sulfate, and/or sodium stearyl fumarate, and/or stearic acid, and/or talc, and/or hydrogenated vegetable oil, and/or combinations thereof.

Solvents can be polar, and/or non-polar, used for manufacturing the immediate-release underlayer, and are selected from the following group: acetone, and/or ethanol, and/or water, and/or benzyl alcohol, and/or combinations thereof.

The extended-release top layer comprises one or more pharmaceutically acceptable excipients selected from diluents, and/or a release-modifying agent, and/or binders, and/or, among others.

The diluent used in the extended-release top layer is selected from the following group: calcium lactate, and/or lactose, and/or microcrystalline cellulose, and/or sodium carboxymethylcellulose, and/or polydextrose, and/or sorbitol, and/or corn starch, and/or sucrose, and/or talc, and/or trehalose, and/or povidone, and/or combinations thereof.

Release-modifying agents used in the extended-release top layer are selected from the following group: gum arabic, and/or acetiltributil citrate, and/or agar, and/or mannitol, and/or methylcellulose, and/or methyl cellulose metolose, and/or pectin, and/or polycarbophil, and/or combinations thereof.

Binders used in the extended-release top layer are selected from the following group: alginic acid, and/or calcium carbonate, and/or calcium lactate, and/or carbopol, and/or carboxymethylcellulose calcium, and/or ceratonia, and/or chitosan, and/or copovidone, and/or dextrose, and/or ethyl cellulose, and/or guar gum, and/or hydroxyethyl methyl cellulose, and/or hydroxypropyl cellulose, and/or hypromellose, and/or anhydrous lactose, and/or sodium alginate, and/or polyvinylpyrrolidone" and/or starch, and/or sucrose, and/or combinations thereof.

Lubricants used in the delayed-release top layer are selected from the following group: calcium phosphate, and/or calcium stearate, and/or hydrogenated castor oil, and/or glyceryl behenate, and/or glyceryl monostearate, and/or lauric acid, and/or leucine, and/or magnesium stearate, and/or myristic acid, and/or polyethylene glycol, and/or polyvinyl alcohol, and/or sodium hyaluronate, and/or sodium lauryl sulfate, and/or sodium stearyl fumarate, and/or stearic acid, and/or talc, and/or hydrogenated vegetable oil, and/or combinations thereof.

Solvents used in the extended-release top layer are selected from acetone, and/or ethanol, and/or water, and/or benzyl alcohol, and/or mixtures thereof.

With the delivery system of the composition described in this invention the number of dosings of ketorolac, and/or their pharmaceutically acceptable salts is reduced, and the duration of analgesic and anti-inflammatory effect in patients is increased, thereby reducing forgetting of timely taking the drug.

The method of preparing the composition generally has the following steps according to each phase.

### Stage 1:

a) Mix the active principle, the diluent(s) and binder(s).
b) The mixture obtained in step a) is granulated with the solvent, is sieved, and follows a drying process and then is sieved again.
c) To the granulate obtained in step b) is added the release modifying agent and is mixed.
d) To the mixture obtained in step c) is added the lubricant and is mixed.
e) It proceeds to compress the mixture obtained in step d) for obtaining what will be the first phase.
f) Optionally, a coating is applied.

### Stage 2:

a) Mix the active principle, the diluent(s) and binder(s).
b) Add ethanol to the above mixture and granulate; after the pellet is obtained, dry and sieve.
c) Add the disintegrant to the granules obtained in b) and mix.
d) Add the lubricant to the mixture obtained in c) and mix.

### Stage 3:

a) Mix the active ingredient, the diluent(s) and/or binder(s).
b) Add alcohol to the mixture obtained and granulate; proceed to dry and sieve.
c) Add the release modifying agent and mix.
d) Add the lubricant and mix.

For obtaining the pharmaceutical composition the core obtained in phase 1 is added to the tableting machine and is compressed together with phase 2 and phase 3.

The following formulations are mentioned by way of example, but not limited to:

### Example 1.

| **Delayed-release core** | |
|---|---|
| **Component** | **Quantity** |
| Ketorolac tromethamine | 2-30 mg |
| Microcrystalline cellulose | 10-20 mg |
| Lactose | 10-20 mg |
| Metolose | 20-60 mg |
| Polyvinylpyrrolidone | 1-20 mg |
| Magnesium stearate | 0.1-10 mg |
| Ethanol | 0.1-10 mg |

| **Immediate-release underlayer** | |
|---|---|
| **Component** | **Quantity** |
| Ketorolac tromethamine | 2-30 mg |
| Microcrystalline cellulose | 15-40 mg |
| Lactose | 12-35 mg |
| Starch | 5-30 mg |
| Polyvinylpyrrolidone | 1-15 mg |
| Crospovidone | 2-19 mg |
| Stearic acid | 0.1-8 mg |
| Ethanol | 0.1-6.5 mg |

| **Extended-release top layer** | |
|---|---|
| **Component** | **Quantity** |
| Ketorolac tromethamine | 5-30 mg |
| Microcrystalline cellulose | 3-17 mg |
| Lactose | 3-18 mg |
| Starch | 6-18 mg |
| Polyvinylpirrolidone | 5-15 mg |
| Metolose | 15-40 mg |
| Stearic acid | 0.1-10 mg |
| Ethanol | 0.1-10 mg |

### Example 2.

| **Delayed-release core** | |
|---|---|
| **Component** | **Quantity** |
| Ketorolac tromethamine | 2-30 mg |
| carboxymethylcellulose | 10-20 mg |
| Sorbitol | 10-20 mg |
| Gum arabic | 20-60 mg |
| Carbopol | 1-20 mg |
| Magnesium stearate | 0.1-10 mg |
| Ethanol-acetone | 0.1-10 mg |

| **Immediate-release underlayer** | |
|---|---|
| **Component** | **Quantity** |
| Drug substance | 2-30 mg |
| carboxymethylcellulose | 15-40 mg |
| Cornstarch | 12-44 mg |
| Povidone | 5-30 mg |
| Hypromellose | 1-15 mg |
| Croscarmellose | 2-19 mg |
| Calcium phosphate | 0.1-8 mg |
| Ethanol-acetone | 0.1-6.5 mg |

| **Extended-release top layer** | |
|---|---|
| **Component** | **Quantity** |
| Drug substance | 5-30 mg |
| carboxymethylcellulose | 3-17 mg |
| Cornstarch | 3-18 mg |
| Povidone | 6-18 mg |
| Hypromellose | 5-15 mg |
| Croscarmellose | 15-40 mg |
| Calcium phosphate | 0.1-10 mg |
| Ethanol-acetone | 0.1-10 mg |

### Example 3.

| **Delayed-release core** | |
|---|---|
| **Component** | **Quantity** |
| Ketorolac tromethamine | 2-30 mg |
| Microcrystalline cellulose | 10-20 mg |
| Lactose | 10-20 mg |
| Metolose | 20-60 mg |
| Polyvinylpyrrolidone | 1-20 mg |
| Magnesium stearate | 0.1-10 mg |
| Ethanol | 0.1-10 mg |

| **Sensory layer** | |
|---|---|
| **Component** | **Quantity** |
| Hydroxypropylmethylcellulose | 2-30 mg |
| Triethylcitrate | 1-10 mg |
| Flavoring | 1-15 mg |
| Water | 5-20 mg |
| Ethanol | 5-20 mg |

| **Immediate-release underlayer** | |
|---|---|
| **Component** | **Quantity** |
| Ketorolac tromethamine | 2-30 mg |
| Microcrystalline cellulose | 15-40 mg |
| Lactose | 12-35 mg |
| Starch | 5-30 mg |
| Polyvinylpyrrolidone | 1-15 mg |
| Crospovidone | 2-19 mg |
| Stearic acid | 0.1-8 mg |
| Ethanol | 0.1-6.5 mg |

| **Extended-release top layer** | |
|---|---|
| **Component** | **Quantity** |
| Ketorolac tromethamine | 5-30 mg |
| Microcrystalline cellulose | 3-17 mg |
| Lactose | 3-18 mg |
| Starch | 6-18 mg |
| Polyvinylpirrolidone | 5-15 mg |
| Metolose | 15-40 mg |
| Stearic acid | 0.1-10 mg |
| Ethanol | 0.1-10 mg |

### Example 4.

| **Delayed-release core** | |
|---|---|
| **Component** | **Quantity** |
| Ketorolac tromethamine | 2-30 mg |
| Microcrystalline cellulose | 10-20 mg |
| Lactose | 10-20 mg |
| Metolose | 20-60 mg |
| Polyvinylpyrrolidone | 1-20 mg |
| Magnesium stearate | 0.1-10 mg |
| Ethanol | 0.1-10 mg |

| **Sensory layer** | |
|---|---|
| **Component** | **Quantity** |
| Hydroxypropylmethylcellulose | 2-30 mg |
| Polyethylene glycol | 1-10 mg |
| Flavoring | 1-15 mg |
| Water | 5-20 mg |
| Ethanol | 5-20 mg |

| **Immediate-release underlayer** | |
|---|---|
| **Component** | **Quantity** |
| Ketorolac tromethamine | 2-30 mg |
| Microcrystalline cellulose | 15-40 mg |
| Lactose | 12-35 mg |
| Starch | 5-30 mg |
| Polyvinylpyrrolidone | 1-15 mg |
| Crospovidone | 2-19 mg |
| Stearic acid | 0.1-8 mg |
| Ethanol | 0.1-6.5 mg |

| **Extended-release top layer** | |
|---|---|
| **Component** | **Quantity** |
| Ketorolac tromethamine | 5-30 mg |
| Microcrystalline cellulose | 3-17 mg |
| Lactose | 3-18 mg |
| Starch | 6-18 mg |
| Polyvinylpirrolidone | 5-15 mg |
| Metolose | 15-40 mg |
| Stearic acid | 0.1-10 mg |
| Ethanol | 0.1-10 mg |

## Claims

1. A solid pharmaceutical composition containing as active ingredient a nonsteroidal anti-inflammatory, which is ketorolac and/or its pharmaceutically acceptable salts; **characterized by** having a triple release system that consists of three phases, which are: a) at least one delayed-release central core containing ketorolac and/or pharmaceutically acceptable salts thereof, and one or more pharmaceutically acceptable excipients; b) an immediate-release underlayer containing ketorolac and/or pharmaceutically acceptable salts thereof, and one or more pharmaceutically acceptable excipients; c) at least one extended-release top layer containing ketorolac and/or pharmaceutically acceptable salts thereof, and one or more pharmaceutically acceptable excipients.

2. A pharmaceutical composition according to claim 1 wherein each of the phases making up the composition contain ketorolac tromethamine and/or pharmaceutically acceptable salts thereof.

3. A pharmaceutical composition according to claims 1 and 2, wherein the nonsteroidal anti-inflammatory is in a concentration of 2 mg to 100 mg, and more preferably 10 mg to 50 mg.

4. A pharmaceutical composition according to claim 1, wherein it comprises a modified release system consisting in a first delayed-release phase, a second immediate-release phase, and a third extended-release phase.

5. A pharmaceutical composition according to claim 1, wherein phase a) comprises a central core of ketorolac and/or their pharmaceutically acceptable salts and at least one pharmaceutically acceptable excipient which is **characterized by** being delayed release.

6. A pharmaceutical composition according to claims 1 and 5 wherein the nucleus is located in the center of the composition.

7. A pharmaceutical composition according to claim 1, wherein the phase b) of the composition comprises an immediate-release underlayer containing ketorolac and/or pharmaceutically acceptable salts thereof and at least one pharmaceutically acceptable excipient.

8. A pharmaceutical composition according to claims 1 and 7, wherein the underlayer may fully or partially cover the core and/or may not contain the core.

9. A pharmaceutical composition according to claim 1, wherein phase c) consists of an extended-release top layer containing ketorolac and/or its pharmaceutically acceptable salts and at least one pharmaceutically acceptable excipient.

10. A pharmaceutical composition according to claims 1 and 9 wherein the top layer may fully and/or partially cover the core, and/or may not contain the core.

11. A pharmaceutical composition according to claims 1 to 10, wherein the pharmaceutically acceptable excipients may be selected from: binders and/or disintegrants and/or diluents and/or lubricants and/or release modifier agents and/or stabilizers and/or solvents and/or surface agents and/or one or more pharmaceutically acceptable excipients.

12. A solid pharmaceutical composition containing as active ingredient a non-steroidal anti-inflammatory, which is ketorolac and/or pharmaceutically acceptable salts thereof; **characterized by** having a triple release system that consists of three phases, which are: a) at least one delayed-release central core containing ketorolac and/or pharmaceutically acceptable salts thereof, and one or more pharmaceutically acceptable excipients, and/or at least a sensory layer; b) an immediate-release underlayer containing ketorolac and/or pharmaceutically acceptable salts thereof, and one or more pharmaceutically acceptable excipients; c) at least one extended-release top layer containing ketorolac and/or pharmaceutically acceptable salts thereof, and one or more pharmaceutically acceptable excipients.

13. A composition according according to claim 12, wherein the sensory layer comprises at least one sweetening, and/or flavoring, and/or coloring, and/or effervescent agent, and/or a coating polymer, and/or plasticizer, and/or solvent, and/or a pharmaceutically acceptable excipient.

14. A composition according to claim 13, wherein the sensory layer allows a patient interaction with the system for ingestion of the composition.

15. Use of the pharmaceutical composition according to claims 1 to 15, for the treatment of mild to moderate pain, which can be altralgia, myalgia, eye pain, photophobia, burning feeling secondary to eye surgery, postoperative pain, neuropathic pain, inflammation, and/or headache.
